# EUROPEAN PATENT APPLICATION

(11) **EP 1 693 067 A1**
(43) Date of publication of application: **23.08.2006**
(21) Application number: 04793770.1
(22) Date of filing: 20.10.2004
(51) Int. Cl.: A61K 39/245, A61K 35/76, A61K 9/02, A61K 39/39, A61K 31/198, A61K 31/787, A61P 31/22

(54) **PHARMACEUTICAL ANTI-HERPETIC COMPOSITION, METHOD FOR PRODUCING A DOSAGE FORM BASED THEREON AND METHOD FOR THE USE THEREOF**

(30) Priority: 30.10.2003 RU 2003131814; 27.09.2004 RU 2004128635
(71) Applicant: Obschestvo S Ogranichennoi Otvetstvennostyu "Rusgen", Moscow 123056 (RU)
(72) Inventor: MUSAEVA, Adilya Rafik kyzy, Moscow, 123022 (RU); BARINSKY, Igor Feliksovich, Moscow, 123060 (RU); LAZARENKO, Alla Arnoldovna, Moscow, 117535 (RU); PETROV, Rem Viktorovich, Moscow, 117334 (RU); KHAITOV, Rakhim Musaevich, Moscow, 123056 (RU); KHAITOV, Musa Rakhimovich, Moscow, 123056 (RU)
(74) Representative: HOFFMANN EITLE
(86) International application number: PCT/RU2004/000414
(87) International publication number: WO 2005/042015

(57) **Abstract**

A new anti-herpetic composition is proposed which comprises a virion preparation containing herpes simplex viruses of serotypes 1 and 2 inactivated by formalin or γ-irradiation; the composition further comprises a hi-tech immunomodulator Polyoxidonium, and also amino acids valine and lysine, as well as a combination consisting of at least two amino acids selected from the group: phenylalanine, leucine, alanine, threonine, histidine, arginine, methionine, and can also comprise isoleucine, which is able to stimulate the formation of anti-herpetic peptides at the cell level. The composition can be formulated into various pharmaceutical forms, and owing to its properties it can be used not only in the case of acute, but also in the case of chronic herpetic pathology.

## Description

### Field of the Art

The present invention relates to the field of immunology and particularly to the provision of a new pharmaceutical anti-herpetic composition, to producing a dosage form based thereon, and to a method for the use thereof.

### Background of the Invention

At present the following chemical preparations are commonly used against the herpes virus: acyclovir, virazol, foscarnet, vidarabine, and others [1]. The corresponding vaccines have a specific activity with regard to the herpes virus. A number of anti-herpetic vaccines, including those based on inactivated virions, have been developed both in Russia and in other countries. They are described, for example, in Russian Federation Patents No. 2014084, 1994, No. 2085582, 1997, No. 2085583, 1997, in US Patents No. 4816250, 1989, No. 5215745, 1993, No. 5602023, 1997.

In the most recent years vaccine anti-herpetic preparations have been developed, in which the genetic structure of the herpes virus is taken into account and which comprise DNA fragments and some other components.

Such are, for example, a recombinant herpes virus vaccine comprising DNA fragments and also peptides which it encodes (US Patent No. 6387376 of May 14, 2002). This vaccine can be used in peritoneal infection. A complex herpes virus vaccine comprising a herpes simplex virus, and also glycoprotein (gp) D and deacylated lipid A monophosphate (Cyprian Application, quoted from identical US Application No. 2002147167 of 10.10.2002).

A vaccine comprising inactivated herpes viruses, predominantly localized in atherosclerotic plaques. This is a polyvalent vaccine comprising HSV-1, HSV-2, HSV-6, human cytomegalovirus, Epstein-Barr virus in combination with a pharmaceutically acceptable carrier or diluent (US Patent 6471965 of 29.10.2002), a recombinant DNA encoding herpes virus glycoproteins: gp50, gpII, gpIII, gpI, HSV gD, bovine herpes virus gI, HV gB, EB VgB, gH, human cytomegalovirus gB (NL Patent 9900034 of 1.10.2003).

A vaccine comprising glycoprotein D2t, against herpes simplex virus, and also adjuvants TH-1, 3-DMPL or aluminum salts (published Application EP 1109574 of 29.04.2003). Also known is a vaccine against herpes simplex virus, comprising peptide epitopes characteristic of HSV (published Application WO 03099860 of 27.11.2003). One of the most recent developments is a live vaccine based on a mutant herpes virus, which is operative in an organism at a temperature of 38.5°C or higher. It is suitable for use in animals (US Patent 2004076642 of 22.04.2004).

From the abovesaid it follows that it is expedient to include immunocompetent substances, such as interferons and antioxidants into complex therapy as is recommended in our previous inventive developments (see, e.g., Russian Federation Patent No. 2142816, 1999), wherein one of the dosage forms convenient for a patient (in the form of a suppository) is also shown. We regard this inventive development as a close analog of the present invention.

Chemotherapeutic agents are not always sufficiently effective, because they do not have specific action with respect to herpes virus only, in principle are polyactive and can be used in other virus diseases.

The above-cited vaccine preparations, though specific and having been prepared on the basis of inactivated and attenuated herpes virus strains, are not all suitable for treating humans. For successfully combating a virus in a highly organized organism, especially in a human organism, it is necessary to take into account humoral and cellular factors, the immune system status, the severity of the virus infection, the stage of disease (latent infection, exacerbation, etc.).

In one's time, experiments with HSV-1 of the subfamily Alphaherpesvirinae had revealed its immunodepressive activity. Inhibition of the T-cell proliferation may be combined with a defect of humoral response to a heterologous antigen. The complicated character of the interaction of the herpes simplex virus, serotype 2 (HSV-2) with the immune system of mice is demonstrated by the fact of enhancement of the host's sensitivity to the infection with Coxsackie B virus, normal response to sheep erythrocytes being preserved. Infection of alveolar or peritoneal macrophages leads to inhibition of their ability to perform the function of antibody-dependent cytotoxicity effectors. In different periods of time after the administration, the virus suppresses the phagocytic activity of the macrophages of the abdominal cavity of mice. At the early stages of the infection a temporary enhancement of the phagocytic activity in alveolar macrophages was determined, followed by suppression of this function. The immunodepressive activity of the virus was investigated by simulating this effect in a culture of peripheral mononuclear cells of human blood. After the infection and appropriate incubation natural killers (NK) were isolated and their cytotoxicity was assessed. In the course of work with the described system it was established that the virus suppresses the cytotoxic function of NK only when monocytes are present in the culture of peripheral blood mononuclear cells. The removal of the monocytes from the system cancels the development of the virus-induced suppression; after the introduction of monocytes the immunologic defect is formed again.-It was also established that the development of one types of suppression conditioned by herpes viruses is realized through monocytes. There are data also about direct effect of the herpes virus on regulatory T-cells, which leads to impairment of the synthesis and of the ability to interact with interleukin-2 [2]

Therefore the described mechanisms of action of the herpes virus on immunocompetent cells and on the immune status as a whole suggest that it is necessary to include into the antivirus compounds those drugs which specifically influence the immune system as well as those drugs which normalize the cell metabolism upon affection of an organism by the herpes simplex virus, especially in cases of sluggish, recurrent and poorly susceptible to medical treatment diseases tending to become chronic, chronic virus infection also belonging to this category.

Notwithstanding the considerable advantages of the above-cited vaccine preparations, certain important aspects of the status of the organism of a patient affected by the herpes virus are not taken into account in said preparations. In patients suffering from herpes, especially in its chronic forms, cellular and humoral dysimmunity is observed, enhanced secretion of glucocorticoid hormones occurs, and the sharp impairment of the metabolism of the virus-affected cells and tissues is observed.

Studies have shown that the choice of an immunomodulating drug and schedule of using thereof in patients is determined by an immunologist, depending on the severity and type of the main disease, concomitant pathology, and also a revealed (sometimes hidden) immunologic defect.

Upon affection of cells of the monocytic-macrophage system, Polyoxidonium, Licopid are used. In the cases of most severe forms of affection in the monocytic-macrophage system, preparations of granulocyte-macrophage colony-stimulating factors Molgramostin (Leucomax), Filgrastim (Neupogen) are used.

In the cases of defects of the cell-mediated immunity, one of the following drugs is used: Polyoxidonium, Tactivinum, Thymoptinum, Thymogenum, Thymalinum. In disturbance of the synthesis of antibodies by B-lymphocytes Myelopidum, Polyoxidonium are used.

Among the preparations with immunostimulating properties three main groups can be distinguished, which are used in practical health care: preparations of microbial origin (Pyrogemalum, Proidigiosanum, Ribomunyl, Sodium nucleate, and others), preparations of endogenous origin: thymus preparations (Tactivinum, Thymalinum, Thymoptinum, Thymactidum, Thymostimulin, and others), preparations of bone-marrow origin (Myelopidum), cytokines (Molgramostin, Reaferonum and others), chemically pure synthetic preparations: therapeutic preparations with revealed immunomodulating properties (for example, Diuciphonum and others), analogs of substances of endogenous origin (Licopid, Thymogenum, and others), really synthetic preparations (Polyoxidonium and others) [3].

It is known that in higher animals the immune system is necessary for combating infectious diseases, that is, the simplest live pathogens: bacteria, microbes, fungi, viruses, and the like. Does immunity exist in invertebrates, for instance, in insects? Searches for an answer to this question have led to discovery of a new class of unique substances.

Insects do not possess an immune system similar to the one mammals have. In insects substances are generated which are capable of blocking alien proteins which got into their organism. However, it has been known long since that insects can combat pathogenic microorganisms. In 1980 a group of researchers headed by Hans Boman of the Stockholm University (Sweden) succeeded in establishing the following: a bacteria-infected solution was injected to a larva of the Hyalophora cecropia moth, and then the substances secreted by the infected larva were collected and analyzed. As a result, the scientists obtained two new chemical compounds - peptide molecules consisting of 35 to 39 amino acids. They were given the name of cecropins. The antimicrobial activity of cecropins proved to be very high [4].

In principle, antimicrobial substances that comprise short molecules consisting of 24 to 40 amino acids have been known long since. More than half a century ago antimicrobial peptides gramicidin and nisin were isolated, which are widely used in pharmaceutical and food industries. Plant antibacterial peptides and peptides from honeybee venom were described long ago. Nevertheless, Boman's discovery provoked interest. For one thing, the isolated peptides at first sight resembled very much the familiar substance melittin contained in honeybee venom, but with one small difference: unlike melittin, cecropins killed bacterial cells of the Escherichia coli type only (so-called gramnegative bacteria) and did not act on other microorganisms or cells of higher organisms.

The opinion predominant among researchers was that antimicrobial peptides are generated by secretory organs of lower animals which have no developed immune system. It was shown that mammals ― rabbits, cows and even humans ― can also secrete similar substances. This occurs predominantly in the region of the intestine, respiratory tract and ureters. Peptides are produced constantly even in the quiescent state of organism, while upon infection or damage of organs a burst in the synthesis of the peptides takes place.

Antimicrobial peptides, though they are slightly inferior to antibiotics in efficiency, act much faster, and, this being the most important, destroy bacteria which are resistant to known antibiotics. However, only those peptides which do not cause hemolysis of mammalian cells can be used in clinics as antibiotics and antifungal agents [5].

The above-said peptides can "do away with" viruses in various ways. Firstly, some of them simply interact with the virus directly, blocking its activity. In such a way they inactivate viruses, e.g., stomatitis virus and even HIV. Secondly, peptides can block the multiplication of HIV virions in an infected organism. Cecropins and melittin act in this way.

### Disclosure of the Invention

The technical problem of the present invention is to provide a new, more effective and progressive complex preparation which would not only specifically act directly on the herpes simplex virus, but would also ensure intensive immunomodulating action on an organism as a whole, mobilize all the organism potentialities for combating virus on the cellular and humoral levels.

The posed problem is solved by that a virion anti-herpetic preparation which contains viruses of herpes simplex of serotypes 1 or 2, inactivated by formalin or γ-radiation, further comprises a hi-tech immunomodulator Polyoxidonium, amino acids valine and lysine, as well as a combination of at least 2 amino acids selected from the group of phenylalanine, leucine, threonine, histidine, arginine, methionine. The pharmaceutical composition has the following proportion of the components:

| | |
|---|---|
| anti-herpetic preparation ― 10⁶ to 10⁷ plaque-forming units/ml of suspension | |
| Polyoxidonium | 0.03―0.06g |
| valine | 0.18―0.25 g |
| lysine | 0.15-0.30 g |
| combination of 2 metabolic amino acids | 0.12- 0.27 g |
| physiological liquid medium | to 100 ml |

As the physiological medium use can be made, e.g., of Eagle's medium for the preparation of vaccines.

The composition may further comprise human albumin as a stabilizer in an amount of 0.22 to 0.24 g per 100 ml, and also a combination of 2―3 water- and fat-soluble vitamins selected from the group of: thiamine, riboflavin, nicotine amide, pyridoxine, ascorbic acid, retinol, tocopherol, or their mixtures in the formulation of the composition in the total amount of from 0.05 to 3.5%.

The effectiveness of treating herpetic infection increases to a considerable extent upon incorporation of *isoleucine* into the composition, by 10―28% as compared to control. Isoleucine may be contained in the formulation of the preparation in an amount of 0.11―0.22 g per 100 ml of composition.

Such a preparation is suitable in treating herpes simplex of types 1 and 2. For this purpose a combination of amino acids and vitamins corresponding to each type of herpes is selected, the doses and administration schedule being selected depending on the patient's condition.

The main subject of the invention is an anti-herpetic composition comprising the following components:
A virion vaccine anti-herpetic preparation - 10⁶ to 10⁷ plaque-forming units/ml of suspension, inactivated by formalin or γ-radiation, Polyoxidonium, and also amino acids valine and lysine, as well as a combination of at least 2 amino acids selected from the group of phenylalanine, leucine, threonine, histidine, arginine, methionine. A detailed description of the new components and their importance for our composition is presented hereinbelow.
The latest-generation domestic immunomodulator - Polyoxidonium (PO) ― is a very efficient immunocompetent agent. PO is a copolymer of 1,4-ethylene-piperazine N-oxide and (N-carboxyethyl)-1,4-ethylene-piperasinium bromide. It is a lyophilized porous mass having a yellowish hue, readily soluble in water, with a molecular mass of 60000 to 100000. In addition to the immunostimulating effect, PO produces pronounced detoxifying, antioxidant and membrane-stabilizing effects.
Lately we have undertaken a number of experiments with the use of PO simultaneously with using the anti-herpetic vaccine applied jointly by parenteral administration. These experiments gave positive results on model infections in laboratory animals (keratitis in rabbits, genital herpes in guinea pigs, meningoencephalitis in mice). The results of these experiments are presented in Table 1.

**Table 1**

| Nos. | Herpes virus vaccines employed | Results of titrating test viruses, lgTCD₅₀/ml | Single-injection dose of Polyoxidonium, mg | Concentration of virus-neutralizing antibodies, neutralization indices |
|---|---|---|---|---|
| 1 | Killed vaccine against herpes simplex virus Type 1 | | - | 2.3 |
| | | 6.5 | 0.1 | 3.75** |
| | | | 0.2 | 3.3* |
| | | | | |
| 2 | Killed vaccine against herpes simplex virus Type 1 | | - | 2.0 |
| | | 5.5 | 0.1 | 3.0* |
| | | | 0.2 | 2.5 |
| | | | | |
| 3 | Killed vaccine against human cytomegalovirus | | - | 2.0 |
| | | 5.0 | 0.1 | 3.0** |
| | | | 0.2 | 2.5 |

| | | | | |
|---|---|---|---|---|
| Note: The significance of difference in the concentrations of virus-neutralizing antibodies in vaccinated animals without use of Polyoxidonium and with the use thereof. **P ≤ 0.01; *P ≤ 0.05. | | | | |

Our investigations have shown that the best effect for the test individuals with different forms of herpes is produced by the hi-tech synthetic drug Polyoxidonium (PO).

In vivo PO produces a more complicated and multiaspect effect on the immune system. Since the development of any immune response begins with cells of the monocytic-macrophage system, and since cytokins produced by monocytes/macrophages have pleiotropic effect, an enhancement of their functional activity under the action of PO leads to the activation of both cellular and humoral immunity. So, in particular, upon introducing PO together with low doses of an antigen a 5 to 10-fold enhancement of the synthesis of antibodies to this antigen over control takes place. It is important to note that such enhancement can be observed in animals with a genetically determined weak reaction to the given antigen. Hence, PO has an ability to bring in motion all factors of organism protection from foreign agents of antigenic nature, and this motion extends in a natural manner so as this occurs in the development of an immune response in an organism. These observations enabled us to choose just Polyoxidonium among the plethora of modern immunostimulators for its successful use in the formulation of a complex pharmaceutical anti-herpetic composition.

We have succeeded to establish empirically that essential amino acids valine and lysine, and also some other amino acids selected by us from a multitude of the known amino acids are very efficient components in the formulation of the composition.

The amino acids employed by us have the following properties apart from the generally known ones:
**Histidine** ― is part of carnosine and anserine, plays an important role in the formation of hemoglobin, is necessary for the generation of erythrocytes. It contributes to controlling the sugar level in blood and to producing energy.
**Leucine** ― is necessary for the growth and healing of bones, muscles. In the metabolism of leucine energy is released, it contributes to stabilization of sugar level in blood. It is found that leucine deficiency in some cases may provoke hypoglycemia, retardation of growth, reduction of bodyweight, changes in kidneys and in the thyroid gland.
**Lysine** - strengthens the immune system, contributes to the growth of bones and formation of collagen, improves attention, has direct anti-herpetic activity. Shortage of lysine in diet leads to disturbance of blood circulation (the number of erythrocytes decreases, the content of hemoglobin in them diminishes). Deficiency may lead to the emaciation of muscles, to disturbance of bone calcification, changes in the liver and lungs, especially in aged and senile patients.
**Methionine** ― is used for the synthesis of choline, is known as a "lipotropic agent", because it reduces the stock of fats in the liver and in an organism as a whole and lowers the amount of cholesterol. Builds up new bone tissue, inhibits onchypathies, protects kidneys and is a natural chelating agent for heavy metals. Methionine is of great importance for the functions of adrenal glands and for the synthesis of adrenalin, this being crucial for metabolic control in virus-affected cells.
**Phenylalanine** - is associated with the function of thyroidal gland and adrenal glands, participates in the formation of a core for the synthesis of thyroxin which is the main hormone of the thyroid gland, participates in the formation if adrenalin. In the organism phenylalanine can be transformed into thyrosine which is used for the synthesis of two main neurotransmitters, dopamine and epinephrine controlling cell metabolism on the neurohumoral level. Phenylalanine is effective in controlling pain and itch sensations. Besides, owing to the secretion of cholecystokinin, phenylalanine has appetite-suppressing action.
**Valine** -valine deficiency may lead to damage of the myeline coating of nerve fibers and to the generation of negative hydrogen balance of the organism, this being extremely dangerous for virus-infected patients.
**Alanine** ― strengthens the immune system, participates in glucose metabolism.
**Arginine** ― shortage of arginine may cause loss of hair, constipations, hepathopathies, and slow healing of wounds, including those caused by damage with virus toxins.
**Isoleucine** possesses a unique property which is used for formulating an antivirus vaccine for the first time ― this amino acid received from the outside (with food) only stimulates the production in human organism (predominantly in the intestine region) of antimicrobial peptides, similar to cecropins which are capable to destroy herpes viruses.

Such a composition, especially supplemented with microelements which enhance immune response to the administration of the preparation, allows using thereof also for more effective combating virus lesions by herpes of serotypes 1 and 2 and some chronic forms of this disease.

A method for using a pharmaceutical anti-herpetic composition by administering thereof to an organism affected by herpes virus is another subject matter of the invention, wherein the above-characterized composition is administered to the organism in an effective dose as a suitable dosage form in a suitable way, selected from the group: perorally, sublingually, intranasally, rectally, vaginally, parenterally, subconjuctivally or in a chewable form.

The action of microelements in the immune system in the case of herpes was studied in detail, using suppositories containing our composition.

The following mechanisms of action of microelements (MEs) exist in the immune system:

### 1. Effect on Specific Receptors

On receptors localized on cytoplasmic membrane: HLA-system, MHC-system (Ni, Cr, Hg).
Adhesins: selectins and integrins (Mn, Hg).
Receptors to transferrin (Al, Ga).
Receptors participating in NK-mediated lysis (Zn).
Cytokine receptors (Zn).
T-cell receptor (Zn, Hg).
Receptors to calcium and magnesium ions (Zn, Mn, Be, Cd, Hg, and others).
Immunoglobulin receptors (Zn).
Receptors localized in intracellular compartments:
   Mitochondria (Fe, Zn), cytoskeleton LIM-proteins (Zn, Se, Li).
Intracellular receptors to calcium on mitochondria, endoplasmic reticulum (Cd, Zn).

### 2. Effect on Enzyme Activity

Many essential MEs are a component of the catalytic site of a number of enzymes. For example, Mn is an essential part of the superoxide dismutase (SOD) of immunocytes, Se forms part of the catalytic site of glutathione peroxidase (isoenzyme VI), Zn is the most important part of numerous Zn-finger proteins controlling the transcription level of other intracellular proteins. The pathways of the action of MEs on the activity of enzymes also exist, which consist in competitive inhibition or allosteric activation of metalloenzymes. For example, Zn is a competitive inhibitor of Ca⁺²⁺, Mg²⁺-dependent endonuclease. This action of Zn has determined its leading role in the immune system as an anti-apoptotic factor.

### 3. Effect on Hormone Activity

- MEs as a component part of hormones.

Zn is a key component of thymosin, a hormone realizing the effects: of the thymus on the T-cell branch of the immune system.

### MEs and Storage of Hormones

Zn, Cr participate in the storage and stabilization of the molecule of insulin which produces multimodulating effect on all insulin-dependent cells of an organism, to which immunocytes belong, too. Zinc provides intracellular storage and stabilization of hormones of neurohypophysis.

### Participation in Hormone Degradation and Elimination

It is known that angiotensin-converting enzyme is Zn-dependent.

Participation in the mechanism of hormone action.

### 4. Effect on Carrier Proteins

Albumins.

Metallothioneins which are synthesized in mononuclear cells of the reticulo-endothelial system of the organism.

Stress proteins, as universal proteins synthesized in cells in response to stress effects (thermal shock, starvation, UV irradiation, effect of heavy metals, chronic infection).

### 5. Physical-Chemical Action of MEs on Immunocyte Membranes

It has been found that, e.g., selenium can produce an antioxidant effect, acting as a cofactor of glutathione peroxidase which provides inactivation of free oxygen species, the generation of which in the immune system ensures both destruction or elimination of a foreign agent (parasite, bacterium), and, in the case of excessive generation of singlet O₂⁻, H₂O₂, ⁻OH, causes damage of the membrane apparatus of the immunocytes themselves. The generation of O₂⁻, H₂O₂, ⁻OH is associated with induction of Haber-Weiss and Fenton reactions under the influence of transition metals (Cu, Zn, Mn, Fe).

Thus, MEs are able through the agency of enzyme and non-enzyme mechanisms of lipid peroxidation (LPO), as well as through the activation of antioxidant mechanisms, to control the physical-chemical properties of cell membranes, including the property of semi-permeability with regard to various biological substrates (antigens, infectious agents, etc.).

### 6. Effect on the presentation, intracellular processing and degradation of antigens (see Item 1).

### 7. Effect on the formation of immunologic memory, and also probably, in the longterm existence of memory cells anti-apoptotic MEs (Zn, Se and others) are involved.

### 8. Effect on the production of immunoglobulins (Zn, Be).

### 9. Effect on the processes of chemotaxis, adhesion and phagocytosis [6, 7].

Numerous tests on laboratory animals have made it possible to select the most effective inicroelements for incorporation thereof into the formulation of the dosage form developed by us (suppository): 2―3 microelements selected from the group: zinc, chromium, selenium and nickel. The presence of said microelements in the formulation of the dosage form enhances the action of the immunomodulator selected by us by 25―30% over the control (without MEs).

Presented below is Table 2, which shows the level of immunologic indices in experimental animals infected with herpes virus, before and after supplementing the formulation of the composition comprising amino acids, including isoleucine with Polyoxidonium.

**Table 2**

| Indices | Before supplementing with Polyoxidonium | After supplementing with Polyoxidonium |
|---|---|---|
| Leucocytes (abs) | 7.6 ± 0.2 | 7.5 ± 0.1 |
| | | p > 0.05 |
| Lymphocytes % | 31.1 ± 1.9 | 31.8 ± 2.3 |
| | | p > 0.05 |
| CD3+% | 58.5 ± 2.2 | 72.1 ± 1.3 |
| | | p < 0.005 |
| CD 4 + % | 30.5 ± 1.1 | 41.4 ± 1.32 |
| | | p < 0.005 |
| CD 8 + % | 18.0 ± 0.5 | 22.8 ± 0.7 |
| | | p < 0.005 |
| CD 16 % | 8.7 ± 1.3 | 11.5 ± 1.2 |
| | | p < 0.05 |
| Ig A mg% | 250 ± 6.5 | 265.2 ± 6.2 |
| | | p > 0.05 |
| Ig G mg% | 1502.1 ± 31.3 | 1575.2 ± 30.5 |
| | | p > 0.05 |
| Ig M mg% | 175.7 ± 9.6 | 182.1 ± 7.8 |
| | | p > 0.05 |
| Neutrophil phagocytosis % | 52.1 ± 2.3 | 67.3 ± 6.1 |
| | | p < 0.005 |

From the Table the effect of PO on the immunologic indices is seen, wherein the cell immunity and the level of immunocompetent proteins significantly increase. It should be noted that the incorporation into the composition of amino acids (valine and lysine, and also isoleucine, and further a combination consisting of at least 2 amino acids selected from the group: phenylalanine, leucine, alanine, threonine, histidine, arginine, methionine) in all the combinations proposed by us accelerated the re-epithelization of damaged tissues (from 15 to 36% over the prototype, see above), depending on the type of the virus. The incorporation of Polyoxidonium into the composition jointly with amino acids not only activated the immunity, which made it possible to prolong considerably the period of remission, to accelerate the process of re-epithelization and healing of skin, and in some cases to essentially succeed in curing some chronic forms of the disease in individual animals (12% of all the individuals under test).

The above-characterized composition can be embodied as dosage forms in which solid, soft or liquid substances can be used as a carrier.

The final form of the composition with the use of solid carriers comprises a tablet, dragee, granule, sachet or powder placed into a capsule.

The final product produced from the composition with the use of liquid carriers comprises a solution, gel, emulsion, suspension, mixture, syrup or liniment.

The composition with the use of soft carriers comprises an ointment, crème, paste, suppository, implant or chewing tablet, or pastille.

The above-said pharmaceutical forms were produced with the help of conventional procedures and auxiliary substances commonly adopted in such cases.

A method of preparing a suppository based on the above-said pharmaceutical composition with the help of a conventional suppository-making technology is a further subject matter of the invention, wherein the active components of the above-described pharmaceutical composition and 2-3 microelements selected from the group: zinc, chromium, selenium and nickel are further introduced into a suppository mass based on cocoa oil by following a conventional technology. A set of microelements as soluble chelate forms is introduced into the formulation of the suppository in an amount of 0.01 to 0.08% based on the total weight.

The results of testing the suppositories thus produced in a group of 76 experimental animals showed that using the new preparation not only activated the immunity, but made it possible to prolong appreciably the period of remission, to accelerate the process of re-epithelization and healing of skin and mucous tissues, and in some cases to essentially succeed in curing of the disease in particular individuals (12% of all the test subjects with the chronic form of the disease).

In our opinion, the incorporation of vitally important ingredients controlling the metabolic processes on all levels, namely, of: PO, valine and lysine, isoleucine stimulating the production of virus-blocking peptides (of cecropin-type) by the intestinal cells, of vitamins of all groups, and also of MEs: zinc, chromium, selenium and nickel into the suppository formulation played an important role in this result.

The invention is supported by particular tests on animals.

*Major performance criteria (over the close analog).* The time of attaining topical convalescence (complete re-epithelization) reduced by 15―36%, the duration of remission increased to 6―7 months on an average, the absence of virus in the smear (PCR-diagnostics) in 98.7% of cases, activation of the antiviral immunity, essentially complete curing in 12% of the follow-ups of the animals:

Among the test animals (herpetic keratitis in rabbits, genital herpes in guinea pigs) 52 individuals were affected with herpes simplex virus of serotypes 1 and 2 in acute form and 24 individuals were affected with chronic form of herpes simplex. The animals with the acute form were divided into three groups: in the 1^{st} group treatment was carried out with the herpetic vaccine described in the prototype; in the 2^{nd} group - with the new pharmaceutical composition; in the control group the animals received chemotherapy in the form of ointments and solutions containing the known anti-herpetic preparations. The group of the animals with the chronic form received new suppositories with a complete set of amino acids, vitamins and microelements.

*Results.* In the 1^{st} group complete re-epithelization took place on the 6^{th} day of treatment on an average, while in the 2^{nd} group complete re-epithelization was noted on the 4^{th} ―5^{th} day of treatment on an average. In the control group complete topical convalescence was stated on the 7^{th} ―10^{th} day from the start of treatment on an average. The virus in control smears was not detected in 95.2% of the 1^{st} group, in 97.8% of the 2^{nd} group, and in 92.5% of the control group. The duration of remission of up to 4 months was observed in 92% of cases in the first group and of up to 6―7 months in 85% of cases in the second group. At the same time, in the control group the duration of remission of up to 4 was observed in 28% only. A highly intensive immune response was registered in the 1^{st} group in 92.1% of cases, while in the 2^{nd} group this was the case in 98.6% with a clear-cut significance (p < 0.01). Thus after the treatment a tendency to an increase in the percentage and absolute counts of natural killer cells, T-lymphocytes of CD8+ phenotypes was noted in the blood serum of the test animals of both groups (in the second group by 11―18% higher) as well as an increase in the virus-induced interferon-α and mitogen-induced interferon-γ production was revealed.

The obtained pharmaceutical suspension taken in an amount of 20 ml is mixed with 0.01-0.03 ml of each of the solutions of salts of 2-3 MEs and cocoa oil to give the total weight of 100 g, from the resulting mass suppositories are prepared by a conventional method, each suppository weighing 0.15―0.20 g. Such suppository contains ≤ 400 µg /g of protein, has a residual humidity ≤ 2.2% and physiological pH value 7.3 ± 0.2, is nontoxic and capable of inducing the synthesis of the virus-neutralizing antibodies in rats, with the neutralization index equal to 3.0 lg TCD₅₀/ml in terms of HSV-1 and to 2.0 lg TCD₅₀/ml in terms of HSV-2, where TCD₅₀ denotes the dose producing cytopathic effect in 50% of test tubes with a cell monolayer, infected with the virus.

Owing to the development of the new highly immunogenic composition, the antigenicity and stability of the specific activity of the preparation are preserved, the anti-herpetic properties of individual organs at the cell level are enhanced, the organism resistance is increased and prolonged not only in acute but also in chronic herpes infection.

### References

1. D.A.Kharkevich "Farmakologiya", GEOTAR-MED, 2000, pp. 549-550.
2. Khaitov R.M. "Immmunogenetika i Immunologiya: Rezistentnost' k Infektsii", Tashkent, 1991.
3. Petrov R.V., Khaitov R.M. "Vaktsiny novogo pokoleniya na osnove sinteticheskikh polionov: istoriya sozdaniya, fenomenologiya i mekhanizmy dejstviya, vnedreniye v praktiku". International Journal on Immmunorehabilitation, 1999, No. 11, pp. 13―36.
4. Khaitoiv R.M., Ignat'eva G.A., Sidorovich I.G., "Immunologiya", Moscow, 2000.
5. Horwitz A.H., Williams R.E., Liu P.-S., Nadell R. "Antimicrob. Agents Chemother.", 1'999, 43, 2314―2316.
6. Skal'nyj A.V. "Mikroelementozy Cheloveka (Diagnostika i Lechenie)", Moscow, 1997.
7. Khaitov R.M. et al., "Ekologicheskaya Immunologiya", Moscow, 1995.

## Claims

1. A pharmaceutical anti-herpetic composition comprising a virion vaccine anti-herpetic preparation containing herpes simplex viruses of serotypes 1 or 2 inactivated by formalin or γ-radiation, and an immunocompetent substance, **characterized in that** it contains Polyoxidonium, valine, lysine, and a combination consisting of at least two metabolic amino acids selected from the group: phenylalanine, leucine, alanine, threonine, histidine, arginine, methionine, with the following proportion of the components:
anti-herpetic preparation ― 10⁶ to 10⁷ plaque-forming units/ml of suspension
| | |
|---|---|
| Polyoxidonium | 0.03―0.06 g |
| valine | 0.18―0.25 g |
| lysine | 0.15―0.30 g |
| combination of metabolic amino acids | 0.12―0.27 g |
| physiological liquid medium | to 100 ml |

2. The composition according to claim 1, **characterized in that** it further comprises an amino acid isoleucine in an amount of 0.11-0.22 g per 100 ml of the composition.

3. The composition according to claim 2, **characterized in that** it further comprises human albumin in an amount of 0.22-0.24 g per 100 ml.

4. The composition according to claim 3, **characterized in that** it further comprises one or more water- and fat-soluble vitamins selected from the group: thiamine, riboflavin, nicotine amide, pyridoxine, ascorbic acid, retinol, tocopherol, or their mixtures in the formulation of the composition in the total amount of from 0.05 to 3.5%.

5. The composition according to claim 4, **characterized in that** it can be formulated into a dosage form in which a solid, soft or liquid substance can be used as a carrier.

6. The composition according to claim 5, **characterized in that** with the use of a solid carrier the final form is a tablet, dragee, granule, sachet or powder placed into a capsule.

7. The composition according to claim 5, **characterized in that** with the use of a liquid carrier the end product is a solution, gel, emulsion, suspension, mixture, syrup or liniment.

8. The composition according to claim 5, **characterized in that** with the use of a soft carrier the end product is an ointment, crème, paste, suppository, implant or chewing tablet, or pastille.

9. A method for preparing a suppository based on the pharmaceutical composition **characterized in** claims 1-4, which method comprises mixing, by following a conventional technology, of the active components and cocoa oil as a carrier, **characterized in that** the composition **characterized in** claims 1―4 and one or more microelements (MEs) selected from the group: zinc, chromium, selenium and nickel are introduced as the active components.

10. The method according to claim 9, **characterized in that** the MEs are introduced as soluble chelate forms in an amount of 0.01―0.08% based on the total weight of the composition.

11. A method for use of the pharmaceutical anti-herpetic composition by administering it to an organism affected by herpes virus, **characterized in that** the composition **characterized in** claims 1-8 is administered to the organism in an effective dose in a suitable dosage form in a suitable way selected from the group: perorally, sublingually, intranasally, rectally, vaginally, parenterally, subconjuctivally or in a chewable form.
